Europäisches Patentamt

European Patent Office

Office européen des brevets

(19)

(11) Veröffentlichungsnummer: **0 279 388**
**B1**

(12) **EUROPÄISCHE PATENTSCHRIFT**

(45) Veröffentlichungstag der Patentschrift:
25.04.90

(51) Int. Cl.⁴: **C07C 317/14**

(21) Anmeldenummer: **88102127.3**

(22) Anmeldetag: **13.02.88**

(54) Verfahren zur Herstellung von 4,4'-dichlordiphenylsulfonen.

(30) Priorität: **17.02.87 DE 3704932**

(43) Veröffentlichungstag der Anmeldung:
**24.08.88 Patentblatt 88/34**

(45) Bekanntmachung des Hinweises auf die Patenterteilung:
**25.04.90 Patentblatt 90/17**

(84) Benannte Vertragsstaaten:
**BE CH DE FR GB IT LI**

(56) Entgegenhaltungen:
**EP-A- 0 062 736
DE-A- 3 306 597**

(73) Patentinhaber: **BASF Aktiengesellschaft,
Carl-Bosch-Strasse 38, D-6700 Ludwigshafen(DE)**

(72) Erfinder: **Schaefer, Gerhard, Dr. Dipl.-Chem.,
Schafrippl 2, D-6900 Heidelberg(DE)**
Erfinder: **Neumann, Peter, Dr, Dipl.-Chem.,
Franz-Schubert-Strasse 1, D-6908 Wiesloch(DE)**

**Beschreibung**

Diese Erfindung betrifft ein neues Verfahren zur Herstellung von 4,4'-Dichlordiphenylsulfon.

4,4'-Dichlordiphenylsulfon ist ein wichtiges Zwischenprodukt, das hauptsächlich für die Herstellung aromatischer Polysulfone und zur Synthese von Bis-(4-aminophenyl)-sulfon, das man sowohl für die Lepratherapie als auch zur Härtung von Epoxidharzen benötigt, verwendet wird. Voraussetzung für diese Einsatzgebiete ist eine hohe Reinheit des 4,4'-Dichlordiphenylsulfons.

Man kann 4,4'-Dichlordiphenylsulfon z.B. nach dem in der DE-PS 1 087 592 beschriebenen Verfahren durch Umsetzung von Chlorbenzol mit einer Mischung aus Schwefeltrioxid und Dimethyl- oder Diethylpyrosulfat herstellen. In der DE-AS 2 252 571 wird die Synthese aus Chlorbenzol und Chlorbenzolsulfonsäure beschrieben. Sie erfordert Temperaturen von 220 bis 260°C, so daß bei überatmosphärischem Druck gearbeitet werden muß.

Die bekannteste Methode zur Herstellung von 4,4'-Dichlordiphenylsulfon ist die nach einer Friedel-Krafts-Reaktion ablaufende Umsetzung von 4-Chlorbenzolsulfochlorid mit Chlorbenzol, wobei man als Katalysator z.B. Eisen-III-chlorid verwendet. Bei dieser Arbeitsweise, die z.B. in der DE-PS 2 704 972 beschrieben wird, ist die Verwendung von Eisenchlorid von Nachteil. Die Umsetzung zum Sulfon wird in Chlorbenzol als Lösungsmittel bei ca. 140°C durchgeführt. Aus J. Am. Chem. Soc. 76, 5491 (1954) ist bekannt, daß Eisen-III-chlorid bei dieser Reaktionstemperatur auch als Chlorierungsmittel auf Chlorbenzol wirkt, so daß das auch als Lösungsmittel eingesetzte und wiedergewonnene Chlorbenzol erhebliche Anteile Dichlorbenzole enthält, die eine aufwendige Aufarbeitung notwendig machen, da auch Dichlorbenzole mit Chlorbenzolsulfochlorid zum Sulfon reagieren können.

Wird die Reaktion ausgehend von Chlorbenzol als Einstufenreaktion ohne Isolierung des Zwischenprodukts Chlorbenzolsulfochlorid durchgeführt, so ist darauf zu achten, daß in der Reaktionsmischung keine Sulfonsäure, kein Thionylchlorid und keine Schwefelchloride (als Verunreingung des Thionylchlorids) mehr vorhanden sind, da die freie Sulfonsäure zur Desaktivierung des Katalysators und Thionylchlorid sowie Schwefelchloride zu unerwünschten Nebenprodukten führen. Erschwerend kommt hinzu, daß man zur vollständigen Umsetzung von Chlorbenzolsulfonsäure mit Thionylchlorid zum entsprechenden Sulfochlorid Dimethylformamid hinzugeben muß, wobei das cancerogene Dimethylcarbamoylchlorid als Nebenprodukt entsteht. Das Eisenchlorid muß aus der Reaktionsmischung durch Hydrolyse entfernt werden. Dabei erhält man einerseits ein sehr korrosives Medium, das hohe Anforderungen an die verwendeten Werkstoffe stellt, andererseits gelangen auf diesem Weg auch bedeutende Chlorbenzolmengen ins Wasser, wodurch eine Aufarbeitung der Abwässer erforderlich wird.

Aufgabe der Erfindung war die Bereitstellung eines Verfahrens zur Herstellung von 4,4'-Dichlordiphenylsulfon aus Chlorbenzol, Schwefeltrioxid oder Chlorsulfonsäure und Thionylchlorid oder Phosgen, bei dem auf die Zugabe der Katalysatoren Eisen-III-chlorid und N,N-Dimethylformamid verzichtet werden kann. Außerdem sollte 4,4'-Dichlordiphenylsulfon in hoher Ausbeute und großer Reinheit erhalten werden.

Diese Aufgabe wurde dadurch gelöst, daß man bei der Herstellung von 4,4'-Dichlordiphenylsulfon durch Erhitzen eines Gemisches aus (a) Chlorbenzol, (b) Chlorsulfonsäure oder Schwefeltrioxid und (c) Thionylchlorid oder Phosgen auf Temperaturen bis 220°C, bezogen auf das Chlorbenzol, mehr als die stöchiometrische Menge an Chlorsulfonsäure oder Schwefeltrioxid und weniger als die stöchiometrische Menge an Thionylchlorid oder Phosgen einsetzt.

Bekanntlich bildet sich bei der chemischen Reaktion von äquimolaren Mengen der Einsatzstoffe (a), (b) und (c) zunächst Chlorbenzolsulfonsäure, die dann zum Chlorbenzolsulfonsäurechlorid chloriert wird, welches für die Bildung von Dichlordiphenylsulfon ein weiteres Mol Chlorbenzol benötigt.

Aufgrund der gewählten nicht-stöchiometrischen Mengenverhältnisse der Einsatzstoffe bildet sich bei der erfindungsgemäßen Umsetzung ein geringer Überschuß an Chlorbenzolsulfonsäure, wodurch die Umsetzung des Chlorbenzolsulfonsäurechlorids zum Sulfon katalysiert wird. Während man bei der üblichen Arbeitsweise, bei der man Chlorbenzol im Überschuß einsetzt, die zur Sulfonbildung erforderliche Temperaturen nicht erreicht, wird beim erfindungsgemäßen Verfahren gewährleistet, daß die Reaktionstemperaturen von 165 bis 220°C erreicht werden. Man erhält schließlich eine Schmelze aus isomeren Dichlordiphenylsulfonen und Chlorbenzolsulfonsäure.

Nach dem Verfahren der Erfindung erhält man besonders vorteilhafte Ergebnisse, wenn man auf 1 Mol Chlorbenzol 0,51 bis 0,63, vorzugsweise 0,54 bis 0,59 Mol Chlorsulfonsäure oder Schwefeltrioxid und 0,49 bis 0,37, vorzugsweise 0,46 bis 0,41 Mol Thionylchlorid oder Phosgen einsetzt.

Man erhitzt das Gemisch der Ausgangsstoffe zur Bildung des 4,4'-Dichlordiphenylsulfons auf 165 bis 220°C, vorzugsweise auf 185 bis 200°C. Zweckmäßigerweise gibt man bis zu 40% höchstens jedoch die Hälfte der insgesamt erforderlichen Chlorbenzolmenge während des Aufheizens des Reaktionsgemisches zu. Die Reaktionsdauer beträgt etwa 1 bis 6 Stunden.

Den Endpunkt der Reaktion bestimmt man zweckmäßigerweise durch Analyse der Reaktionsmischung auf Chlorbenzolsulfonsäurechlorid. Diese Analyse kann z.B. durch DC, GC, HPLC erfolgen. Bei Verwendung der Gaschromatographie kann man auch die Menge des zugesetzten Chlorbenzols überwachen und diese so dosieren, daß bei Reaktionsende die Mengen an Chlorbenzol und Chlorbenzolsulfonsäurechlorid deutlich unter 1 % liegen, wodurch ein vollständiger Umsatz des eingesetzten Chlorbenzols erreicht wird.

Die Aufarbeitung der Schmelze, die die Isomeren des Dichlordiphenylsulfons etwa in der Zusammensetzung 91 % 4,4'-; 6 % 3,4'- und 3 % 2,4'- enthält, kann man z.B. nach an sich bekannten Methoden vornehmen. Nach einer neuen, besonders vorteilhaften Ausführungsform des erfindungsgemäßen Verfahrens, bei der man das gewünschte 4,4'-Isomere in hoher Ausbeute und Reinheit isoliert, wird die Schmelze mit Alkoholen oder aromatischen Lösungsmitteln, gegebenenfalls unter Druck, verdünnt, wobei das 4,4'-Dichlordiphenylsulfon ausfällt. Als Alkohole sind z.B. Alkanole mit 1 bis 4 C-Atomen und als aromatische Lösungsmittel z.B. Chlorbenzol, Toluol geeignet. Besonders vorteilhafte Ergebnisse verhält man, wenn man als Fällmittel Methanol oder Chlorbenzol verwendet. Nach einer bevorzugten Arbeitsweise erfolgt die Verdünnung der Schmelze durch Eindosieren in vorgelegtes Methanol, wobei man die Temperatur so wählt, daß ein Druck von etwa 4 bis 6 bar erreicht wird.

Das zum Ausfällen verwendete Lösungsmittel läßt sich aus der Mutterlauge durch Destillation leicht zurückgewinnen. Die im Destillationsrückstand verbliebene Chlorbenzolsulfonsäure kann als Katalysator in die Reaktion zurückgeschleußt werden. Bei Verwendung von Chlorbenzol als Fällmittel kann man die erhaltene Mutterlauge direkt zur Reaktion wiederverwenden. Da durch diese Mutterlauge die zur Katalyse notwendige Menge an Chlorbenzolsulfonsäure in die Reaktion zurückgelangt, sind zum Folgeansatz stöchiometrische Mengen an Thionylchlorid und Chlorsulfonsäure zu geben.

Nach dem erfindungsgemäßen Verfahren, das auch kontinuierlich durchgeführt werden kann, erhält man 4,4'-Dichlorphenylsulfon in hoher Ausbeute und Reinheit. Es ist dabei von erheblichem Vorteil, daß die Umsetzung zum Chlorbenzolsulfonsäurechlorid nicht vollständig sein muß, so daß man auf eine Katalyse dieser Reaktion mit Dimethylformamid verzichten kann. Günstig ist es auch, daß an die Reinheit des Thionylchlorids in Bezug auf Schwefelchloride keine hohen Anforderungen gestellt werden müssen, da diese unter den Reaktionsbedingungen nicht zur Bildung von unerwünschten Nebenprodukten, wie Dichlordiphenylsulfide, führen.

Beispiel 1

Zu 1125 g (10 mol) Chlorbenzol gibt man bei 55 bis 65°C 629 g (5,4 mol) Chlorsulfonsäure und 547,4 g (4,6 mol) Thionylchlorid. Nach Beendigung der HCl-Entwicklung wird die Reaktionsmischung aufgeheizt. Bei 135°C beginnt der Ansatz zu sieden und dabei Salzsäure zu entwickeln. Nach 4 bis 5 h läßt der Rückfluß merklich nach und die Innentemperatur steigt auf 195°C an. Ist diese Temperatur erreicht, so ist kein Chlorbenzolrückfluß mehr feststellbar. Durch GC-Analytik des Ansatzes stellt man fest, daß das Chlorbenzolsulfonsäurechlorid nahezu vollständig zum Sulfon umgesetzt ist (<1 %).

Der gesamte Ansatz wird auf 150°C abgekühlt und unter Siedekühlung in 1 Liter Methanol eingerührt. Nach dem Abkühlen wird abfiltriert und mit Methanol gewaschen. Man erhält 944 g (71,5 % d.Th. bez. auf Thionylchlorid) 4,4'-Dichlordiphenylsulfon mit einem Schmelzpunkt von 149 bis 150°C. Die GC-Analyse des Produkts ergibt folgende Werte:
4,4'-Isomeres $\geq$ 99,7 %
3,4'-Isomeres < 0,1 %
2,4'-Isomeres $\leq$ 0,2 %.

Beispiele 2 bis 7

Das Verfahren aus Beispiel 1 wird wiederholt, wobei die Ausgangsstoffe jedoch in den aus der Tabelle ersichtlichen Mengen eingesetzt werden. Man erhält 4,4'-Dichlordiphenylsulfon in den in der Tabelle angegebenen Ausbeuten und Reinheit.

| Beispiel | $CLSO_3H$ | $SOCl_2$ | Chlorbenzol | Reak.-dauer | Ausb. % bez. | Ausb. bez. | Gehalt an 4,4'-Isom. |
|---|---|---|---|---|---|---|---|
| Nr. | (mol) | (mol) | (mol) | (h) | auf $SOCl_2$ | auf Chlorb. | (GC) |
| 1 | 5,4 | 4,6 | 10 | 18 | 71,5 | 63,5 | 99,7 |
| 2 | 5,2 | 4,8 | 10 | 32 | 56,3 | 53,6 | 99,55 |
| 3 | 5,4 | 4,6 | 10 | 26 | 62,2 | 57,8 | 99,6 |
| 4 | 5,5 | 4,5 | 10 | 20 | 70,8 | 64,3 | 99,6 |
| 5 | 5,7 | 4,3 | 10 | 17 | 72,1 | 62,6 | 99,7 |
| 6 | 5,8 | 4,2 | 10 | 15 | 73,4 | 61,2 | 99,7 |
| 7 | 6 | 4 | 10 | 14 | 74,5 | 59,6 | 99,7 |

Beispiel 8

Zu 787,5 g (7 mol) Chlorbenzol gibt man bei 55 bis 65°C 629 g (5,4 mol) Chlorsulfonsäure und 547,4 g (4,6 mol) Thionylchlorid und heizt diese Mischung zügig bis auf 185°C auf. Hierbei tritt kein merklicher Rückfluß auf. Man erhitzt weiter und gibt bei 195°C weitere 337,5 g (3 mol) Chlorbenzol in dem Maße zu, daß die Innentemperatur nicht merklich absinkt. Die weitere Aufarbeitung erfolgt wie in Beispiel 1 beschrieben. Man erhält 953 g (72,2 %, bezogen auf Thionylchlorid) 4,4′-Dichlordiphenylsulfon mit einem Schmelzpunkt von 149 bis 150°C.

Beispiele 9 bis 15

Man verfährt analog Beispiel 8, wobei man jedoch die 337,5 g Chlorbenzol zum Reaktionsgemisch gibt, wenn dieses die in der Tabelle angegebene Reaktionstemperatur aufweist.

| Beispiel | Chlorbenzol-zugabe | Reaktionsdauer | Restgehalt an 4-Chlor-benzolsulfonsäurechl. | Ausb. bez. auf Thionylchlorid | Farbe des Produkts |
|---|---|---|---|---|---|
| Nr. | bei °C | h | % lt. GC | % | |
| 8 | 195 | 17 | 0,3 | 72,2 | weiß |
| 9 | 200 | 16 | 0,2 | 73 | weiß |
| 10 | 210 | 15,5 | 0,2 | 73,3 | grau |
| 11 | 220 | 14,5 | nicht detektierbar | 73,5 | grau |
| 12 | 190 | 18 | 0,3 | 72,1 | weiß |
| 13 | 185 | 19 | 1 | 70,6 | weiß |
| 14 | 175 | 25 | 3 | 68,8 | weiß |
| 15 | 165 | 40 | 8 | 62,3 | weiß |

Beispiel 16

Man führt die durch Destillation vom Methanol befreite Mutterlauge aus Beispiel 1 in das Reaktionsgefäß zurück, fügt 562,5 g (5 mol) Chlorbenzol hinzu und erzeugt durch Zugabe von 476 g (4 mol) Chlorsulfonsäure und 466 g (4 mol) Thionylchlorid das Chlorbenzolsulfonsäurechlorid. Nach dem Aufheizen des Ansatzes auf 195°C gibt man 337,5 g (3 mol) Chlorbenzol in der Geschwindigkeit dazu, daß die Temperatur nicht absinkt. Nach der Aufarbeitung des Ansatz analog Beispiel 1 erhält man 850 g (74 % d.Th.) Dichlordiphenylsulfon mit einem Schmelzpunkt von 149 bis 150°C. Die GC-Analytik ergibt folgende Werte:
4,4′-Isomeres = 99,6 %
2,4′-Isomeres = 0,3 %
3,4′-Isomeres = 0,1 %.

Beispiel 17

Man verfährt analog Beispiel 8 und arbeitet wie folgt auf: Die Reaktionsschmelze läßt man in ein Druckgefäß, in dem 1,5 l Methanol vorgelegt sind, einlaufen und heizt auf 110 bis 115°C auf. Dabei stellt sich ein Druck von 4,5 bar ein. Man rührt unter diesen Bedingungen noch 1 Stunde nach. Nach dem Abkühlen wird abfiltriert und mit Methanol gewaschen. Man erhält 931 g Dichlordiphenylsulfon der Zusammensetzung:
4,4′-Isomer = 99,8 %
2,4′-Isomer ≦ 0,1 %
3,4′-Isomer ≦ 0,1 %

**Patentansprüche**

1. Verfahren zur Herstellung von 4,4′-Dichlordiphenylsulfon durch Erhitzen eines Gemisches aus (a) Chlorbenzol, (b) Chlorsulfonsäure oder Schwefeltrioxid und (c) Thionylchlorid oder Phosgen auf Temperaturen bis 220°C, dadurch gekennzeichnet, daß man, bezogen auf das Chlorbenzol, mehr als die stöchiometrische Menge an Chlorsulfonsäure oder Schwefeltrioxid und weniger als die stöchiometrische Menge an Thionylchlorid oder Phosgen einsetzt.

2. Verfahren nach Anspruch 1, dadurch gekennzeichnet, daß man, bezogen auf 1 Mol Chlorbenzol, 0,51 bis 0,63 Mol Chlorsulfonsäure oder Schwefeltrioxid und 0,49 bis 0,37 Mol Thionylchlorid oder Phosgen einsetzt.

3. Verfahren nach Anspruch 1, dadurch gekennzeichnet, daß man, bezogen auf 1 Mol Chlorbenzol 0,54 bis 0,59 Mol Chlorsulfonsäure oder Schwefeltrioxid und 0,46 bis 0,41 Mol Thionylchlorid oder Phosgen einsetzt.

4. Verfahren nach Anspruch 1, dadurch gekennzeichnet, daß man einen Teil der Chlorbenzolmenge, höchstens jedoch die Hälfte, im Laufe des Erhitzens zum Umsetzungsgemisch gibt.

5. Verfahren nach Anspruch 1, dadurch gekennzeichnet, daß man die bei der Umsetzung erhaltene Schmelze mit Alkoholen oder aromatischen Lösungsmitteln versetzt und das beim Abkühlen ausfallende 4,4'-Dichlordiphenylsulfon abtrennt.

6. Verfahren nach Anspruch 5, dadurch gekennzeichnet, daß man als Alkohole Alkanole mit 1 bis 4 C-Atomen und als aromatische Lösungsmittel Chlorbenzol oder Toluol verwendet.

7. Verfahren nach Anspruch 5, dadurch gekennzeichnet, daß man die nach Abtrennung des 4,4'-Dichlordiphenylsulfons erhaltene Mutterlauge nach destillativer Rückgewinnung des Lösungsmittels und nach Zugabe der drei Ausgangsstoffe erneut für eine Umsetzung gemäß Anspruch 1 heranzieht.

## Claims

1. A process for preparing 4,4'-dichlorodiphenyl sulfone by heating a mixture of (a) chlorobenzene, (b) chlorosulfonic acid or sulfur trioxide and (c) thionyl chloride or phosgene at up to 220°C, which comprises using, based on the chlorobenzene, a more than stoichiometric amount of chlorosulfonic acid or sulfur trioxide and a less than stoichiometric mount of thionyl chloride or phosgene.

2. A process as claimed in claim 1, wherein, based on 1 mole of chlorobenzene, from 0.51 to 0.63 mole of chlorosulfonic acid or sulfur trioxide and from 0.49 to 0.37 mole of thionyl chloride or phosgene are used.

3. A process as claimed in claim 1, wherein, based on 1 mole of chlorobenzene, from 0.54 to 0.59 mole of chlorosulfonic acid or sulfur trioxide and from 0.46 to 0.41 mole of thionyl chloride or phosgene are used.

4. A process as claimed in claim 1, wherein some of the chlorobenzene, but not more than half, is added to the reaction mixture in the course of the heating.

5. A process as claimed in claim 1, wherein the melt obtained in the course of the reaction is mixed with an alcohol or an aromatic solvent and the 4, 4'-dichlorodiphenyl sulfone which precipitates on cooling is separated off.

6. A process as claimed in claim 5, wherein the alcohol used is an alkanol of from 1 to 4 carbon atoms and the aromatic solvent used is chlorobenzene or toluene.

7. A process as claimed in claim 5, wherein the mother liquor left behind on removal of the 4,4'-dichlorodiphenyl sulfone is reused for a reaction as set forth in claim 1 after the solvent has been recovered by distillation and after the three starting materials have been added.

8. A process as claimed in claim 5, wherein the melt is admixed with chlorobenzene and the mother liquor left behind after the 4,4'-dichlorodiphenyl sulfone is separated off is reused for a reaction as set forth in claim 1 after sulfur trioxide or chlorosulfonic acid and thionyl chloride or phosgene have been added.

## Revendications

1. Procédé de préparation de 4,4'-dichlorodiphényl-sulfone par chauffage d'un mélange de (a) chlorobenzène, (b) acide chlorosulfonique ou anhydride sulfurique et (c) chlorure de thionyle ou phosgène, à des températures allant jusqu'à 220°C, caractérisé en ce que on met en réaction, par rapport au chlorobenzène, plus que la quantité stoechimoétrique d'acide chlorosulfonique ou d'anhydride sulfurique et moins que la quantité stoechiométrique de chlorure de thionyle ou de phosgène.

2. Procédé selon la revendication 1, caractérisé en ce qu'on met en réaction, pour 1 mole de chlorobenzène, 0,51 à 0,63 mole d'acide chlorosulfonique ou d'anhydride sulfurique et 0,49 à 0,37 mole de chlorure de thionyle ou de phosgène.

3. Procédé selon la revendication 1, caractérisé en ce qu'on met en réaction, pour 1 mole de chlorobenzène, 0,54 à 0,59 mole d'acide chlorosulfonique ou d'anhydride sulfurique et 0,46 à 0,41 mole de chlorure de thionyle ou de phosgène.

4. Procédé selon la revendication 1, caractérisé en ce qu'on ajoute au mélange réactionnel, au cours du chauffage, une partie de la quantité de chlorobenzène, toutefois au plus la moitié.

5. Procédé selon la revendication 1, caractérisé en ce qu'on ajoute des alcools ou des solvants aromatiques à la masse fondue fournie par la réaction et on sépare la 4,4'-dichlorodiphénylsulfone précipitée par refroidissement.

6. Procédé selon la revendication 5, caractérisé en ce qu'on utilise comme alcools des alcanols de 1 à 4 atomes de carbone et comme solvants aromatiques du chlorobenzène ou du toluène.

7. Procédé selon la revendication 5, caractérisé en ce qu'on introduit à nouveau pour une réaction selon la revendication 1, après récupération par distillation du solvant et après addition des trois substances de départ, la liqueur mère obtenue après séparation de la 4,4'-dichlorodiphénylsulfone.

8. Procédé selon la revendication 5, caractérisé en ce qu'on ajoute aux masses fondues du chlorobenzène et on introduit à nouveau pour une réaction selon la revendication 1 la liqueur mère obtenue après séparation de la 4,4′-dichlorodiphénylsulfone, après addition d'anhydride sulfurique ou d'acide chlorosulfonique et de chlorure de thionyle ou de phosgène.